# EUROPEAN PATENT SPECIFICATION

(11) **EP 1 025 895 B1**
(45) Date of publication and mention of the grant of the patent: **28.12.2005**
(21) Application number: 98947831.8
(22) Date of filing: 12.10.1998
(51) Int. Cl.: A61L 9/20, A61L 9/22, B01D 53/88, B03C 3/38

(54) **AIR CLEANER**
LUFTREINIGER
EPURATEUR D'AIR

(30) Priority: 14.10.1997 JP 28086397
(43) Date of publication of application: 09.08.2000
(73) Proprietor: DAIKIN INDUSTRIES, LIMITED, Osaka-shi, Osaka 530-0015 (JP)
(72) Inventor: KAKIMOTO, Akihisa, Kanaoka-kojo, Sakai-seisakusho, Sakai-shi, Osaka 591-8022 (JP); ITOH, Yoshihide, Kanaoka-kojo, Sakai-seisakusho, Sakai-shi, Osaka 591-8022 (JP); NUNOKAWA, Syunichi, Kanaoka-kojo, Sakai-seisakusho, Sakai-shi, Osaka 591-8022 (JP); KATO, Toshiyuki, Kanaoka-kojo, Sakai-seisakusho, Sakai-shi, Osaka 591-8022 (JP); ODA, Yasuhiro, Kanaoka-kojo, Sakai-seisakusho, Sakai-shi, Osaka 591-8022 (JP)
(74) Representative: Steil, Christian
(86) International application number: PCT/JP1998/004597
(87) International publication number: WO 1999/019052

(56) References cited:
- JP-A- 4 197 418
- JP-A- 8 121 827
- JP-A- 9 206 628
- JP-A- 10 061 986
- PATENT ABSTRACTS OF JAPAN vol. 1997, no. 10, 31 October 1997 (1997-10-31) -& JP 09 141054 A (MATSUSHITA SEIKO CO LTD), 3 June 1997 (1997-06-03)
- PATENT ABSTRACTS OF JAPAN vol. 1997, no. 07, 31 July 1997 (1997-07-31) -& JP 09 066096 A (USHIO INC), 11 March 1997 (1997-03-11)
- PATENT ABSTRACTS OF JAPAN vol. 1998, no. 14, 31 December 1998 (1998-12-31) -& JP 10 234835 A (TOSHIBA CORP;TOSHIBA AVE CORP), 8 September 1998 (1998-09-08)

## Description

The present invention relates to an air cleaner for removing dust or the like in air.

An air cleaner of an electric dust collection type has been conventionally known. In this type of air cleaner, dust in an air current is ionized upon taking an ion shower in an ionization section, and the ionized dust is adsorbed by a dust collection section arranged on the downstream side of the ionization section in the direction of the air current.

The ionization section has a plurality of longitudinal ionization wires and pairs of opposite electrode plates each opposite to each other with the ionization wire interposed therebetween. On the other hand, examples of the dust collection section arranged on the downstream side of the ionization section include one obtained by alternately laminating cathode plates and anode plates, and a sheet-shaped electrostatic filter.

In the conventional dust collection section, however, dust can be removed, while an odorous component cannot be removed.

In recent years, there has been provided an apparatus for carrying a photocatalyst in a carrying member and irradiating the photocatalyst with light including ultraviolet rays to remove an odorous component, for example.

It is considered that, as the carrying member for carrying the photocatalyst, a honeycomb-shaped structure having a lot of vent holes along the direction of an air current, for example, is used because an area where it is brought into contact with the air current must be as large as possible.

In this case, a light source lamp for irradiating the photocatalyst carried in the honeycomb-shaped structure with light including ultraviolet rays is arranged in such a state as to irradiate light in a direction approximately parallel to the direction of the air current because it must illuminate an inner surface of each of the vent holes.

However, a part of the light from the light source lamp passes through the vent holes, so that the light is not effectively utilized. As a result, a request for a higher cleaning capability cannot be coped with.

From Patent Abstracts of Japan, vol. 1997, no. 07, 31 July 1997 (1997-07-31) & JP 09 066096A (Ushio Inc.), 11 March 1997 (1997-03-11) an air cleaner according to the preamble of claim 1 has become known comprising two honeycomb bodies containing photo excitation catalyst materials being arranged at an angle with the flow direction of the air. Between the honeycomb bodies an lamp is arranged for irradiating the inside of the vent holes with rays.

In view of this it is an object of the invention to provide an improved air cleaner capable of effectively utilizing light from a light source to achieve a high cleaning capability.

This object is achieved by an air cleaner according to claim 1.

In this construction, the light from the light source lamp is irradiated onto the honeycomb-shaped photocatalyst section, and a part of the irradiated light is irradiated onto the sheet-shaped photocatalyst section upon passing through the vent holes of the honeycomb-shaped photocatalyst section.
Consequently, it is possible to effectively utilize the light from the light source for cleaning by the photocatalyst. As a result, it is possible to achieve a high cleaning capability.

In a preferred embodiment of the present invention, the sheet-shaped photocatalyst section is laminated on a surface, on the downstream side in the direction of the air current, of a sheet-shaped electrostatic filter, to construct a laminated filter.

In this construction, the electrostatic filter for collecting dust and the photocatalyst filter for deodorization or the like are laminated and integrated with each other. Therefore, the air cleaner is easy to handle.

In this case, it is preferable that the air cleaner further comprises a contact assisting member for bringing the sheet-shaped electrostatic filter into contact with a grounding member. Consequently, charge can be prevented from being stored in the sheet-shaped electrostatic filter, thereby making it possible to prevent the dust collecting capability of the sheet-shaped electrostatic filter from being decreased.

Furthermore, the air cleaner may further comprise an ionization section for charging contaminant particles before leading to the sheet-shaped electrostatic filter by inducing discharges between an ionization wire and a grounding electrode plate arranged opposite thereto. In this case, the grounding member may be a conductive member for supporting the grounding electrode plate. Consequently, the charge on the sheet-shaped electrostatic filter can be removed in simple construction. The conductive member may be formed integrally with the grounding electrode plate.

Furthermore, it is preferable that the contact assisting member is carried in a reinforcing member for reinforcing the honeycomb structure (for example, is formed integrally with the reinforcing member) and is arranged such that the laminated filter can be pressed against the grounding member. Consequently, the contact assisting member can be supported in simple construction, thereby making it possible to reliably bring the sheet-shaped electrostatic filter into contact with the grounding member in simple construction.

It is preferable that the laminated filter is composed of a roll filter capable of renewing a used area portion pulled out into the air current.

In this construction, when the electrostatic filter and the photocatalyst filter in a used area portion of the roll filter become dirty, they can be collectively renewed. Therefore, the air cleaner is easy to maintain.

The sheet-shaped photocatalyst section may be joined to a surface, on the upstream side in the direction of the air current, of the honeycomb-shaped photocatalyst section.

In this construction, the sheet-shaped photocatalyst and the honeycome-shaped photocatalyst section can be collectively handled. Therefore, the air cleaner is easy to handle.

The foregoing and other objects, features and effects of the present invention will become more apparent from the following description of the present invention when taken in conjunction with the accompanying drawings.

### Brief Description of Drawings

Fig. 1 is an exploded perspective view of an air cleaner as an embodiment of the present invention;
Fig. 2 is an exploded perspective view of a principal part of an ionization section in the air cleaner shown in Fig. 1;
Fig. 3 is a horizontal sectional view of a principal part of the air cleaner shown in Fig. 1; and
Fig. 4 is a horizontal sectional view of a photocatalyst element in an air cleaner as another embodiment of the present invention.

### Modes for Carrying out the Invention

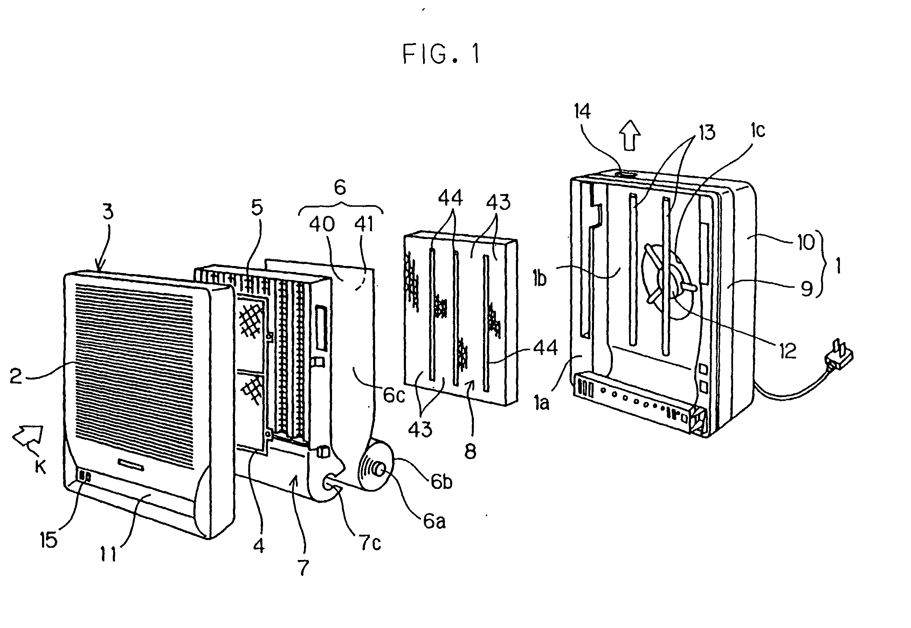

Fig. 1 is an exploded perspective view of an air cleaner including an ionization section according to an embodiment of the present invention. Referring to Fig. 1, in the air cleaner, a foremost part of an air cleaner main body 1 is covered with a front panel 3 provided with a suction grill 2. Air is sucked into the air cleaner main body 1 through the suction grill 2.

A recess 1b is formed on a front surface 1a of the air cleaner main body 1. A pre-filter 4 for removing relatively large refuge or dust, a filter case 7 for carrying a mechanism for removing the dust, and a photocatalyst element 8 for cleaning contaminants are contained in the recess 1b so as to be attachable or detachable. The filter case 7 has an ionization section 5 for inducing discharges for charging contaminant particles mounted on its front surface and has a roll filter 6 including an electrostatic filter 40 serving as a dust collection section mounted on its rear surface. The photocatalyst element 8 constitutes a honeycomb-shaped photocatalyst section having a photocatalyst for cleaning contaminants upon being irradiated with ultraviolet rays carried on its surface or in its inner part.

The front surface 1a of the air cleaner main body 1 is constituted by a front casing 9. The recess 1b is formed in the front casing 9. The air cleaner main body 1 is constructed by combining a rear casing 10 serving as a base casing in a box shape with the rear of the front casing 9.

An opening 1c is formed near the center of the recess 1b. An air fan 12 mounted on the rear casing 10 and causing room air to circulate through the roll filter 6, is exposed from the opening 1c. One or a plurality of light source lamps 13 for irradiating light including ultraviolet rays toward the photocatalyst element 8 are mounted on the recess 1b. As the light source lamp 13, a straight pipe-type cathode-ray tube for emitting light having a wavelength of 320 to 420 nanometers, for example, can be illustrated.

The room air sucked in from the suction grill 2 is cleaned by passing through the pre-filter 4, the ionization section 5, the roll filter 6, the photocatalyst element 8 and the air fan 12, and is then brown off from a blow-off louver 14 provided on the top of the air cleaner main body 1.

A lower part of the front panel 3 is constructed as an operation display panel 11 comprising various types of operation switches and various types of display units. An air inlet 15 is provided at one end of the operation display panel 11. The room air is supplied through the air inlet 15 to a dust detecting sensor (not shown) serving as a contaminant detecting sensor. Air sucked in from the air inlet 15 is released outward from an air outlet (not shown) provided on the top of the rear casing 10 successively through the dust detecting sensor and a P plate containing chamber (not shown).

The roll filter 6 is constructed as a laminated filter obtained by laminating a photocatalyst filter 41 serving as a sheet-shaped photocatalyst section on a rear surface of the electrostatic filter 40 (a side surface on the downstream side in the direction of an air current), as described in detail later. The roll filter 6 comprises a roll 6b wound around a core 6a, and a used area portion 6c held in the filter case 7 in a state where it is pulled out into the air current K from the roll 6b. The core 6a is held in a groove-shaped core holding section 7c of the filter case 7 so as to be attachable and detachable.

Furthermore, the photocatalyst element 8 is constructed by connecting a plurality of honeycomb structures 43 to one another through reinforcing members 44 made of resin.

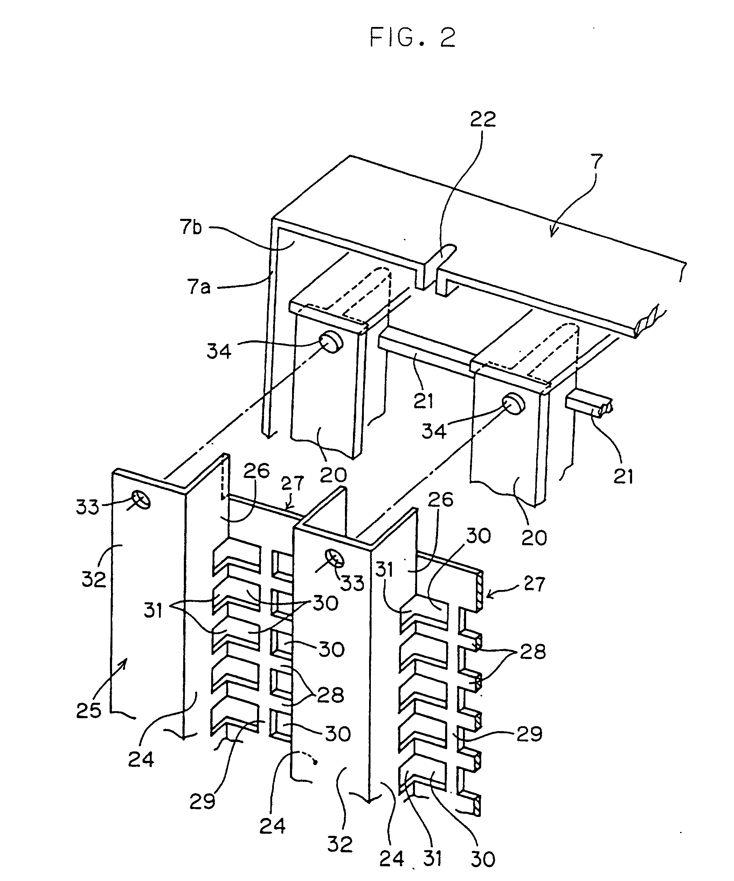

Fig. 2 is an exploded perspective view of a principal part of the ionization section. The filter case 7 is composed of a resin frame whose whole is integrally formed. A recess 7b is formed on a front surface 7a of the filter case 7, and a plurality of vertical frames 20 each having a T shape in cross section are arranged parallel to one another in the recess 7b. Both ends of the adjacent vertical frames 20 are connected to each other by a horizontal frame 21. Reference numeral 22 denotes a supporting groove for supporting an end of an ionization wire.

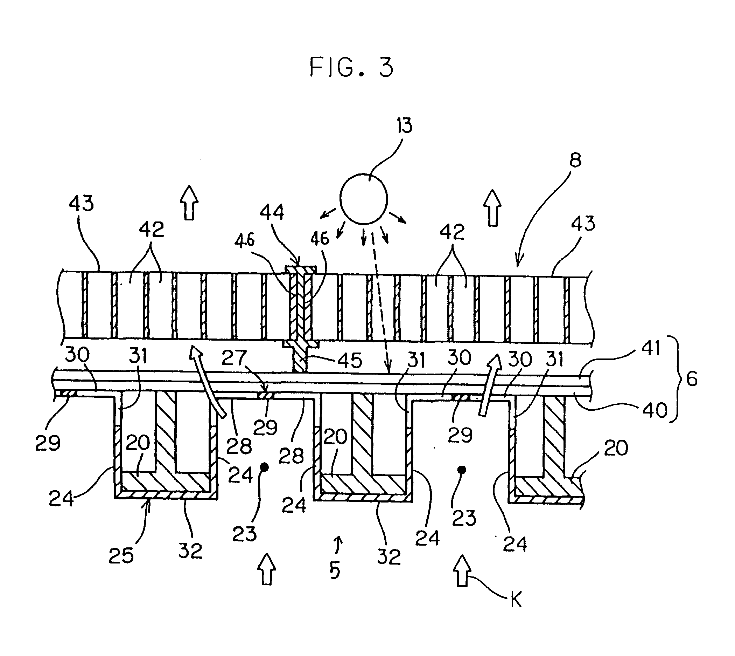

Fig. 3 is a schematic horizontal sectional view of a member for performing an air cleaning function. Referring to Figs. 2 and 3, the ionization section 5 is arranged on the upstream side in the direction of the air current of the roll filter 6 serving as a dust collection section, to ionize dust in the air current. The ionization section 5 comprises a plurality of ionization wires 23 and an opposite electrode plate forming member 25 constructed by connecting a plurality of sets of opposite electrode plates 24 arranged opposite to each other with the ionization wire 23 interposed therebetween.

The opposite electrode plate forming member 25 has a conductive lattice-shaped portion 27 integrally formed between edges 26, on the downstream side in the direction of the air current, of the opposite electrode plates 24 in each of the sets. The respective opposite electrode plates 24, which are adjacent to each other, in the adjacent sets are connected to each other by a web 32, to have a channel shape in cross section. The opposite electrode plate forming member 25 has obtained its entire shape by subjecting a steel plate such as SGCC to sheet metal formation.

The conductive lattice-shaped portion 27 comprises a lot of horizontal frames 28 horizontally extending so as to connect the adjacent opposite electrode plates 24 to each other and arranged spaced a predetermined distance apart from each other, and one or a plurality of vertical frames 29 for connecting respective central parts of the horizontal frames 28 to one another (one vertical frame 29 in the present embodiment). By the horizontal frames 28 and the vertical frame 29, the conductive lattice-shaped portion 27 has a lot of openings 30, through which the air current is to be passed, provided side by side therein.

Openings 31 are also formed at an end, on the downstream side in the direction of the air current, of each of the opposite electrode plates 24. The opening 31 of the opposite electrode plate 24 extends so as to connect with the opening 30 in the conductive lattice-shaped portion 27. A part of an air duct is constituted by the openings 30 and 31. Reference numeral 33 denotes a hole in which a positioning projection 34 on the side of the filter case 7 is to be fitted.

Referring to Fig. 3, the roll filter 6 is composed of a multi-functional laminated filter obtained by joining an electrostatic filter 40 arranged on its front surface on the upstream side in the direction of the air current for collecting dust and a sheet-shaped photocatalyst filter 41 arranged on a side surface (a rear surface) on the downstream side in the direction of the air current of the electrostatic filter 40 and carrying a photocatalyst. The electrostatic filter 40 and the photocatalyst filter 41 can be joined to each other by thermal fusion using thermal fusion powder, for example, or a needle punch.

It is possible to use, as the electrostatic filter 40, a conductive non woven fabric such as a polyolefin series non woven fabric. The electrostatic filter 40 is abutted against the conductive lattice-shaped portion 27, to release unnecessary charge, which may be stored by collecting dust, through the conductive lattice-shaped portion 27.

The photocatalyst element 8 is formed by carrying a photocatalyst in a plurality of honeycomb-shaped structures 43 serving as a photocatalyst carrying member defining an array of a lot of vent holes 42 along the air current K. An example of the honeycomb structure 43 is one formed by alternately laminating flat plates and waveform-shaped plates. An example of the flat plate is vinyl chloride resin impregnated with titanium oxide, described later.

A photocatalyst means a material which absorbs light such as ultraviolet rays and applies its energy to a reactant to cause a chemical reaction. Examples of main functions of the photocatalyst include ① a deodorization function by removal of an odorous component, ② a function of decomposing contaminants which are not an odorous component, and ③ a function of sterilizing microbes and inactivating viruses (a so-called bacterial/antibacterial function). These functions are achieved by an oxidation-decomposition function of the photocatalyst.

An example of a photocatalyst having an oxidation-decomposition function is titanium oxide (TiO₂) having an anatase-type crystal structure. Titanium oxide having an anatase-type crystal structure is preferable in that it can exhibit a high cleaning capability even by weak ultraviolet rays. Other examples include zinc oxide (ZnO) and tungsten oxide (WO₃).

The reinforcing member 44 is composed of a resin molded product, for example. The reinforcing member 44 has a pair of groove-shaped portions 46 which open in opposite directions and are formed so as to vertically extend. A corresponding edge of the photocatalyst element 8 is fitted in and fixed to each of the groove-shaped portions 46 with adhesives. In the reinforcing member 44, a projection 45 projecting toward the roll filter 6 is formed so as to vertically extend. The projection 45 performs the function of reliably bringing the electrostatic filter 40 in the roll filter 6 into contact with the conductive lattice-shaped portion 27. Further, the reinforcing member 44 prevents a load from being applied to the light source lamp 13 by the backward deflection of the photocatalyst element 8 at the time of maintenance, for example.

According to the present embodiment, light from the light source lamp 13 is irradiated onto the photocatalyst element 8 in a honeycomb shape, and the light passing through the vent hole 42 of the photocatalyst element 8 is irradiated onto the photocatalyst filter 41 in a sheet shape positioned ahead, as indicated by an arrow of broken line in Fig. 3. Consequently, the light from the light source lamp 13 can be effectively utilized for cleaning by a photocatalyst, thereby making it possible to achieve a high cleaning capability.

The electrostatic filter 40 for collecting dust and the sheet-shaped photocatalyst filter 41 for deodorization or the like are laminated and integrated with each other. Therefore, the air cleaner is easy to handle. Moreover, the integrated filters are taken as the roll filter 6. When the electrostatic filter 40 and the photocatalyst filter 41 in the used area portion 6c become dirty, therefore, they can be collectively renewed. Therefore, the air cleaner is superior in maintenance.

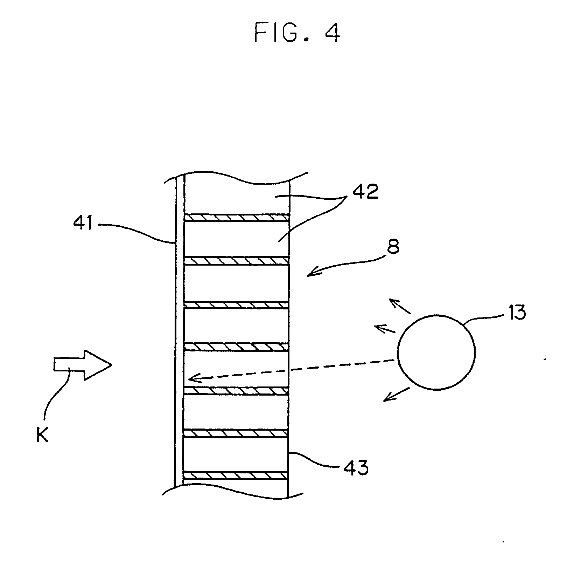

Fig. 4 illustrates a photocatalyst element of an air cleaner according to another embodiment of the present invention. Referring to Fig. 4, the present embodiment differs from the embodiment shown in Fig. 1 in that the photocatalyst filter 41 serving as a sheet-shaped photocatalyst section is contained in the roll filter 6 in the embodiment shown in Fig. 1, while a photocatalyst filter 41 is joined to a surface, on the upstream side in the direction of an air current, of a photocatalyst element 8 in the present embodiment.

In the present embodiment, a cleaning capability can be improved by effective utilization of light, as in the embodiment shown in Fig. 1. In addition, the photocatalyst filter 41 serving as a sheet-shaped photocatalyst section and the photocatalyst element 8 serving as a honeycomb-shaped photocatalyst section can be collectively handled. Therefore, the air cleaner is easy to handle.

The present invention is not limited to the above-mentioned embodiments. Although in each of the embodiments, the photocatalyst filter 41 serving as a sheet-shaped photocatalyst section is held in the roll filter 6 or the photocatalyst element 8 serving as a honeycomb-shaped photocatalyst section, the sheet-shaped photocatalyst filter can be also independently held.

Although the embodiments of the present invention have been described, the present invention can be embodied in other embodiments. Various design changes can be made within the scope of the terms in the claims.

### Industrial Applicability

As described in the foregoing, an air cleaner according to the present invention is used for removing dust or the like in air.

## Claims

1. An air cleaner, comprising:
a honeycomb-shaped photocatalyst section (8) formed with a photocatalyst for cleaning contaminants upon being irradiated with ultraviolet rays carried in a honeycomb structure (43) defining an array of a plurality of vent holes (42) in the direction of an air current (K); and
a light source lamp (13) for irradiating light including ultraviolet rays toward the honeycomb-shaped photocatalyst section (8);
**characterized by**
a sheet-shaped photocatalyst section (41) formed with a photocatalyst for cleaning contaminants upon being irradiated with ultraviolet rays carried on a sheet perpendicular to the direction of an air current (K), the sheet-shaped photocatalyst section (41) and the honeycomb-shaped photocatalyst section (8) being arranged in this order;
a part of the light from the light source lamp (13) being irradiated onto the sheet-shaped photocatalyst section (41) upon passing through the vent holes (42) of the honeycomb-shaped photocatalyst section (8); and
by a contact assisting member (45) for bringing a sheet-shaped electrostatic filter (40) into contact with a grounding member (27);
wherein said sheet-shaped photocatalyst section (41) is laminated on a surface, on the downstream side in the direction of the air current, of said sheet-shaped electrostatic filter (40), to construct a laminated filter (6).

2. The air cleaner of claim 1, **characterized by** an ionization section (5) for charging contaminant particles before leading to the sheet-shaped electrostatic filter (40) by inducing discharges between an ionization wire (23) and a grounding electrode plate (24) arranged opposite thereto,
the grounding member (27) being a conductive member (27) for supporting the grounding electrode plate (24).

3. The air cleaner of claim 1 or 2, **characterized in that** the contact assisting member (45) is carried in a reinforcing member (44) for reinforcing the honeycomb structure (43) and is arranged such that the laminated filter (6) can be pressed against the grounding member (27).

4. The air cleaner of any one of claims 1 to 3, **characterized in that** the laminated filter (6) is composed of a roll filter (6) capable of renewing a used area portion (6c) pulled out into the air current (K).

5. The air cleaner of any one of claims 1 to 3, **characterized in that** the sheet-shaped photocatalyst section (41) is joined to a vent surface of the honeycomb-shaped photocatalyst section (8).

## Patentansprüche

1. Luftreiniger mit:
einem wabenförmigen Lichtkatalysator-Abschnitt (8), der mit einem Lichtkatalysator zum Reinigen von Kontaminationen bei Bestrahlung mit ultravioletten Strahlen in einer wabenförmigen Struktur (43) ausgebildet ist, die ein Array aus einer Mehrzahl von Belüftungslöchern (42) in der Richtung eines Luftstromes (K) definiert, und
einer Lichtquellenlampe (13) zur Bestrahlung des wabenförmigen Lichtkatalysator-Abschnittes (18) mit Licht, das ultraviolette Strahlen enthält,
**gekennzeichnet durch**
einen folienförmigen Lichtkatalysator-Abschnitt (41), der mit einem Lichtkatalysator zum Reinigen von Kontaminationen bei Bestrahlung mit ultravioletten Strahlen ausgebildet ist und auf einer Folie getragen ist, die senkrecht zu der Richtung eines Luftstroms (K) ist, wobei der folienförmige Lichtkatalysator-Abschnitt (41) und der wabenförmige Lichtkatalysator-Abschnitt (8) in dieser Reihenfolge angeordnet sind;
wobei ein Teil des Lichtes aus der Lichtquellenlampe (13) auf den folienförmigen Lichtkatalysator-Abschnitt (41) gerichtet ist, nachdem dieses **durch** die Belüftungslöcher (42) des wabenförmigen Lichtkatalysator-Abschnittes (8) gelangt ist, und
**durch** ein kontaktunterstützendes Element (45), um ein folienförmiges elektrostatisches Filter (40) in Kontakt mit einem Erdungselement (27) zu bringen;
wobei der folienförmige Lichtkatalysator-Abschnitt (41) auf der stromabwärtigen Seite in der Richtung des Luftstromes des folienförmigen elektrostatischen Filters (40) auf eine Oberfläche laminiert ist, um ein laminiertes Filter (6) zu bilden.

2. Luftreiniger nach Anspruch 1, **gekennzeichnet durch** einen Ionisationsabschnitt (5), um kontaminierende Partikel zu laden, bevor sie zu dem folienförmigen elektrostatischen Filter (40) geführt werden, indem Entladungen zwischen einem Ionisationsdraht (23) und einer Erdungselektrodenplatte (24), die dazu gegenüberliegend angeordnet ist, erzeugt werden,
wobei das Erdungselement (27) ein leitfähiges Element (27) ist, um die Erdungselektrodenplatte (24) zu halten.

3. Luftfilter nach Anspruch 1 oder 2, **dadurch gekennzeichnet, dass** das kontaktunterstützende Element (45) in einem verstärkten Element (44) gehalten ist, um die Wabenstruktur (43) zu verstärken, und derart angeordnet ist, dass das laminierte Filter (6) gegen das Erdungselement (27) gedrückt werden kann.

4. Luftfilter nach irgendeinem der Ansprüche 1 bis 3, **dadurch gekennzeichnet, dass** das laminierte Filter (6) aus einem Rollenfilter (6) besteht, das in der Lage ist, einen gebrauchten Flächenbereich (6c), der aus dem Luftstrom (K) herausgezogen ist, zu erneuern.

5. Luftreiniger nach irgendeinem der Ansprüche 1 bis 3, **dadurch gekennzeichnet, dass** der folienförmige Lichtkatalysator-Abschnitt (41) mit einer Belüftungsfläche des wabenförmigen Lichtkatalysator-Abschnitts (8) verbunden ist.

## Revendications

1. Purificateur d'air, comprenant :
une section de photocatalyseur en forme de nid d'abeilles (8) comportant un photocatalyseur pour éliminer des impuretés lorsqu'il est irradié avec des rayons ultraviolets supporté dans une structure en nid d'abeilles (43) définissant un réseau d'une pluralité d'évents (42) dans la direction d'un courant d'air (K) ; et
une lampe formant source de lumière (13) pour rayonner une lumière comprenant des rayons ultraviolets vers la section de photocatalyseur en nid d'abeilles (8) ;
**caractérisé par** :
une section de photocatalyseur en forme de feuille (41) comportant un photocatalyseur pour éliminer des impuretés lorsqu'il est irradié avec des rayons ultraviolets supporté sur une feuille perpendiculaire à la direction d'un courant d'air (K), la section de photocatalyseur en forme de feuille (41) et la section de photocatalyseur en forme de nid d'abeilles (8) étant agencées dans cet ordre ;
une partie de la lumière provenant de la lampe formant source de lumière (13) étant rayonnée sur la section de photocatalyseur en forme de feuille (41) lors de son passage à travers les évents (42) de la section de photocatalyseur en forme de nid d'abeilles (8) ; et
par un élément d'assistance de contact (45) pour amener un filtre électrostatique en forme de feuille (40) en contact avec un élément de mise à la masse (27) ;
dans lequel ladite section de photocatalyseur en forme de feuille (41) est déposée sur une surface, du côté aval dans la direction du courant d'air, dudit filtre électrostatique en forme de feuille (40), pour réaliser un filtre stratifié (6).

2. Purificateur d'air selon la revendication 1, **caractérisé par** une section d'ionisation (5) pour charger des particules d'impuretés avant de les mener vers le filtre électrostatique en forme de feuille (40) en induisant des décharges entre un fil d'ionisation (23) et une plaque d'électrode de mise à la masse (24) agencée face à celui-ci,
l'élément de mise à la masse (27) étant un élément conducteur (27) pour supporter la plaque d'électrode de mise à la masse (24).

3. Purificateur d'air selon la revendication 1 ou 2, **caractérisé en ce que** l'élément d'assistance de contact (45) est supporté dans un élément de renforcement (44) pour renforcer la structure en nid d'abeilles (43) et est agencé de sorte que le filtre stratifié (6) puisse être pressé contre l'élément de mise à la masse (27).

4. Purificateur d'air selon l'une quelconque des revendications 1 à 3, **caractérisé en ce que** le filtre stratifié (6) est composé d'un filtre en rouleau (6) capable de renouveler une partie de surface utilisée (6c) sortie dans le courant d'air (K).

5. Purificateur d'air selon l'une quelconque des revendications 1 à 3, **caractérisé en ce que** la section de photocatalyseur en forme de feuille (41) est unie à une surface d'évents de la section de photocatalyseur en forme de nid d'abeilles (8).
